# EUROPEAN PATENT SPECIFICATION

(11) **EP 3 106 516 B1**
(45) Date of publication and mention of the grant of the patent: **05.06.2024**
(21) Application number: 14882402.2
(22) Date of filing: 25.04.2014
(51) Int. Cl.: C12N 1/21, C12N 15/52, C12P 13/08, C07K 14/34

(54) **RECOMBINANT MICROORGANISM OF GENUS ESCHERICHIA WITH L-THREONINE PRODUCTIVITY, AND METHOD FOR PRODUCING L-THREONINE USING SAME**
REKOMBINANTER MIKROORGANISMUS DER GATTUNG ESCHERICHIA MIT L-THREONINPRODUKTIVITÄT UND VERFAHREN ZUR PRODUKTION VON L-THREONIN DAMIT
MICRO-ORGANISME DE RECOMBINAISON DE GENRE ESCHERICHIA À PRODUCTIVITÉ DE L-THRÉONINE ET PROCÉDÉ DE PRODUCTION L-THRÉONINE L'UTILISANT

(30) Priority: 12.02.2014 WO PCT/KR2014/001154
(43) Date of publication of application: 21.12.2016
(73) Proprietor: CJ Cheiljedang Corporation, Seoul 04560 (KR)
(72) Inventor: KIM, Hyung Joon, Seoul 08226 (KR); KWON, Su Yon, Suwon-si Gyeonggi-do 16509 (KR); KOH, Eun Sung, Suwon-si Gyeonggi-do 16493 (KR); LEE, Ji Sun, Yongin-si Gyeonggi-do 16874 (KR); LEE, Keun Cheol, Hwaseong-si Gyeonggi-do 18427 (KR); HWANG, Young Bin, Seoul 08576 (KR); HONG, Hyeong Pyo, Gangneung-si Gangwon-do 25528 (KR)
(74) Representative: Dehns
(86) International application number: PCT/KR2014/003649
(87) International publication number: WO 2015/122569

(56) References cited:
- KR-B1- 101 058 894
- KR-B1- 101 145 943
- US-A1- 2006 257 979
- US-B2- 7 332 310
- STEFFEN N. LINDNER ET AL: "Impact of a new glucose utilization pathway in amino acid-producing Corynebacterium glutamicum", BIOENGINEERED BUGS, vol. 2, no. 5, 1 September 2011 (2011-09-01), pages 291-295, XP055123920, ISSN: 1949-1018, DOI: 10.4161/bbug.2.5.17116
- CARSTEN BÄUMCHEN ET AL: "Myo-inositol facilitators IolT1 and IolT2 enhance d-mannitol formation from d-fructose in Corynebacterium glutamicum", FEMS MICROBIOLOGY LETTERS, vol. 290, no. 2, 8 January 2009 (2009-01-08), pages 227-235, XP055327736, GB ISSN: 0378-1097, DOI: 10.1111/j.1574-6968.2008.01425.x
- DONG, XUNYAN ET AL.: 'Metabolic engineering of Escherichia coli and Corynebacterium glutamicum for the production of L-threonine' BIOTECHNOLOGY ADVANCES vol. 29, no. 1, 03 August 2010, ISSN 0734-9750 pages 11 - 23, XP027555933

## Description

### Technical Field

The present invention relates to a recombinant microorganism of the genus *Escherichia* having enhanced rate of producing L-threonine, which is obtained by transformation so as to contain a permease of *Corynebacterium* origin represented by SEQ ID NO: 1 or SEQ ID NO: 2, or a permease having at least 80% identity to SEQ ID NO: 1 or SEQ ID NO: 2, and to a method of producing L-threonine using the recombinant microorganism.

### Background Art

L-threonine, a kind of essential amino acid, is widely used as an additive to animal feed and food, and as fluids and synthetic materials for medical and pharmaceutical use. L-threonine is mainly produced by fermentation using *Escherichia coli, Serratia, Providencia* or *Corynebacterium,* developed by artificial mutation methods or gene recombination methods, or artificial mutant strains thereof. Genes related to the biosynthesis of threonine and various methods for increasing the expression of these genes have been developed, but the demand for a method capable of producing L-threonine in high yield in a more cost-effective manner still exists.

It is known that GalP protein that is encoded by *galP* in *E. coli* is galactose permease that transports a variety of monosaccharides, including galactose and glucose, into cells (V. Hernandez-Montalvo F. Valle F. Bolivar G. Gosset, Appl Microbiol Biotechnol (2001) 57:186-191). In addition, it is known that the GalP protein also acts as glucose permease (Venter, Henrietta et al., Biochemical Journal (2002) 363:243-252). It was reported that, when the expression of the galP gene in *E. coli* is increased, for example, by increasing the copy number of the gene, the production of threonine in the *E*. *coli* is increased (WO 2004/087937).

It was reported that inositol permease that is encoded by *iolT1* and *iolT2* genes in *Corynebacterium glutamicum* can also act as glucose permease (Ikeda et al., Appl Microbiol Biotechnol (2011) 90:1443-1451). It was also reported that *iolT1* and *iolT2* genes have high homology with the galP gene of *E. coli.* However, the correlation between inositol permease and threonine production has not yet been reported.

Steffen N. Lindner et al., (2011) Bioengineered Bugs 2(5):291-295 discloses the effect on L-lysine yield of oxerexpressing inositol permeases iolT1 and iolT2 and a glucokinase Glk or PpgK in PTS-deleted C. glutamicum.

Carsten Bäumchen et al., (2009) FEMS Microbiology Letters 290(2): 227-235 discloses the cloning of genes iolT1 and IolT2 from C. glutamicum ATCC13032.

The present inventors have found that, when *iolT1* gene and/or *iolT2* gene encoding inositol permease in microorganisms of the genus *Corynebacterium* is introduced into a microorganism of the *Escherichia Coli*, the microorganism of the genus *Escherichia* has enhanced rate of producing L-threonine.

### Disclosure

### Technical Problem

Therefore, it is an object of the present invention to provide a recombinant microorganism of the genus *Escherichia* having enhanced rate of producing L-threonine, which is obtained by transformation so as to contain a permease of *Corynebacterium* origin represented by SEQ ID NO: 1 or SEQ ID NO: 2, or a permease having at least 80% identity to SEQ ID NO: 1 or SEQ ID NO: 2.

Another object of the present invention is to provide a method of producing L-threonine comprising the steps of:
inoculating and culturing the recombinant microorganism of the invention and separating L-amino acid from the culture.

### Technical Solution

In order to accomplish the above object, the present invention provides a recombinant microorganism and method as defined in the appended claims.

### Advantageous Effects

According to the present invention, the growth rate of a strain is greatly increased compared to those of conventional strains so that the strain can produce L-threonine in high yield. Thus, the production of industrially significant L-threonine can be greatly increased.

### Description of Drawings

FIG. 1 shows the homology alignment of the amino acid sequences of the *galP* of *E. coli* origin and the *iolT1* and *iolT2* of *Corynebacterium glutamicum* origin.
FIG. 2 shows a cleavage map of the recombinant vector pCC1BAC-*iolT1* comprising the *iolT1* gene of *Corynebacterium glutamicum* origin.
FIG. 3 shows a cleavage map of the recombinant vector pCC1BAC-*iolT2* comprising the *iolT2* gene of *Corynebacterium glutamicum* origin.
FIG. 4 shows a cleavage map of the recombinant vector pCC1BAC-*iolT1-iolT2* comprising the *iolT1* and *iolT2* genes of *Corynebacterium glutamicum* origin.

### Mode for Invention

The present invention provides a recombinant microorganism of genus *Escherichia* having enhanced rate of producing L-threonine, which is obtained by transformation so as to contain a permease of *Corynebacterium* origin represented by SEQ ID NO: 1 or SEQ ID NO: 2, or a permease having at least 80% identity to SEQ ID NO: 1 or SEQ ID NO: 2.

In the present invention, the permease of *Corynebacterium* origin may be of *Corynebacterium glutamicum* origin, and preferably *Corynebacterium glutamicum* ATCC 13032 origin, but is not limited thereto.

Most preferably, the permease of *Corynebacterium* origin may have an amino acid sequence represented by SEQ ID NO: 1 or SEQ ID NO: 2. The permease of *Corynebacterium* origin having the amino acid sequence of SEQ ID NO: 1 is encoded by the *iolT1* gene having a nucleotide sequence represented by SEQ ID NO: 3, and the permease of *Corynebacterium* origin having the amino acid sequence of SEQ ID NO: 2 is encoded by the *iolT2* gene having a nucleotide sequence represented by SEQ ID NO: 4.

In addition, mutants having a mutation in the amino acid sequences of the above proteins, or permeases having a homology of at least 80%, preferably at least 90%, more preferably at least 95%, and particularly preferably at least 97%, to the amino acid sequences of the above proteins, are also included in the scope of the present invention, as long as they are proteins having permease activity as disclosed in the present invention.

As used herein, the term "homology" refers to the identity between two amino acid sequences. The homology can be determined using methods well known to those skilled in the art, for example, BLAST 2.0 which calculates parameters such as score, identity or similarity.

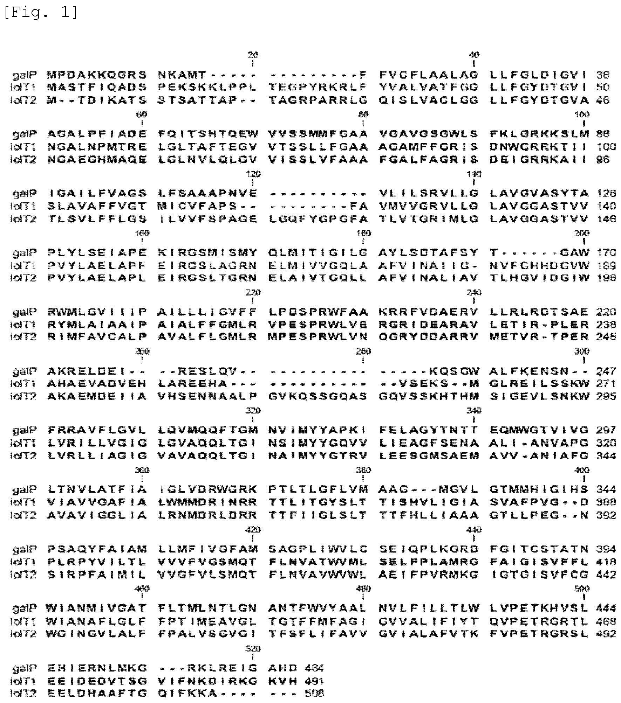

In an example of the present invention, genes having homology to the *galP* gene that encodes glucose permease in *E*. *coli* were identified from *Corynebacterium glutamicum* ATCC 13032. As a result, the amino acid sequence encoded by *iolT1* (NCBI Reference Sequence: NC_006958.1, cg0223) derived from *Corynebacterium glutamicum* ATCC 13032 showed a homology of 34% to the amino acid sequence of *E. coli galP,* and the amino acid sequence encoded by *iolT2* (NCBI Reference Sequence: NC_006958.1, cg3387) showed a homology of 31% to the amino acid sequence of the *E. coli galP* (see FIG. 1).

The *E. coli galP* is known, and can be obtained from the *E*. *coli* genome sequence (Accession no. AAC75876) described in Blattner et al., Science 277: 1453-1462 (1997), and can also be obtained from databases such as the National Center for Biotechnology Information (NCBI) database and the DNA Databank of Japan (DDBJ) database.

The recombinant microorganism of the genus *Escherichia* according to the present invention may be *E. coli* or an L-threonine-producing *E. coli* mutant. More preferably, the recombinant microorganism of the genus *Escherichia* is *E. coli* KCCM 10541 (Korean Patent No. 10-0576342) derived from *E. coli* KFCC 10718 (Korean Patent No. 10-0058286) . *E. coli* KCCM 10541 is an L-threonine-producing strain that has a methionine auxotroph phenotype, resistance to a threonine analogue, resistance to a lysine analogue, resistance to an isoleucine analogue, and resistance to a methionine analogue, and comprises two or more copies of phosphoenol pyruvate carboxylase gene (ppc gene) and threonine operon introduced into the chromosome.

A method that enables the permease-encoding gene to be expressed in the microorganism of the genus *Escherichia* is not specifically limited. For example, in order to enable the expression of the permease-encoding gene, a recombinant vector comprising the permease-encoding gene may be transformed into a microorganism, or the copy number of the permease-encoding gene may be increased, or an expression regulatory sequence of the permease-encoding gene may be modified. In addition, two or more of these methods may also be used in combination. Generally, methods for increasing the expression level of the related to threonine biosynthesis-related gene include a method of increasing the copy number of the gene in a single microorganism. For this, a plasmid whose copy number is maintained at a high level is used (Sambrook et al., Molecular cloning, 2nd edition, 1989, 1.3-1.5). Specifically, a desired gene is inserted into a plasmid whose copy number is maintained at a high level, and the resulting recombinant plasmid is transformed into a microorganism. In this case, the effect of increasing the copy number of the gene to the copy number of the plasmid per microorganism can be obtained. In addition, a method of inserting the threonine biosynthesis-related gene into chromosomal DNA may also be used.

As used herein, the term "vector" refers to a DNA construct containing the nucleotide sequence of a target protein-encoding gene operably linked to a suitable regulatory sequence so as to be able to express the target gene in a suitable host cell. The regulatory sequence includes a promoter capable of initiating transcription, any operator for regulating this transcription, a sequence encoding a suitable mRNA ribosome binding site, and a sequence for regulating the termination of transcription and translation. Once transformed into a suitable host, the vector may replicate or function independently of the host genome, or may integrate into the genome itself.

The vector that is used in the transformation is not specifically limited and may be any vector known in the art, as long as it can replicate in a host. Examples of the commonly used vectors may include natural or recombinant plasmids, cosmids, viruses, and bacteriophages.

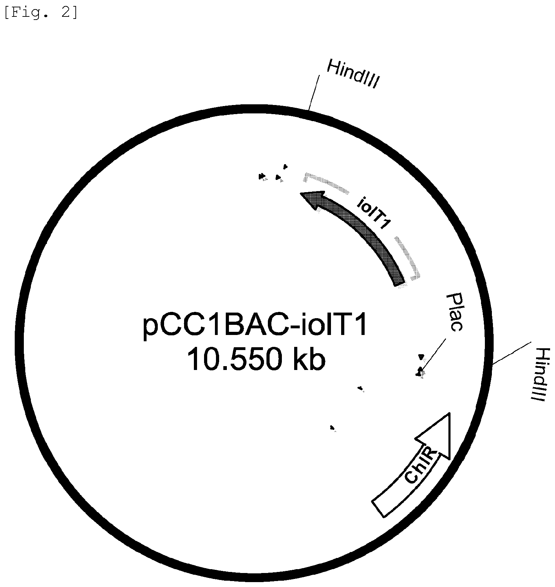

A recombinant vector comprising the *iolT1* gene of *Corynebacterium glutamicum* origin may be used. Preferably, the recombinant vector is pCC1BAC-*iolT1.* More preferably, the recombinant vector has a cleavage map shown in FIG. 2.

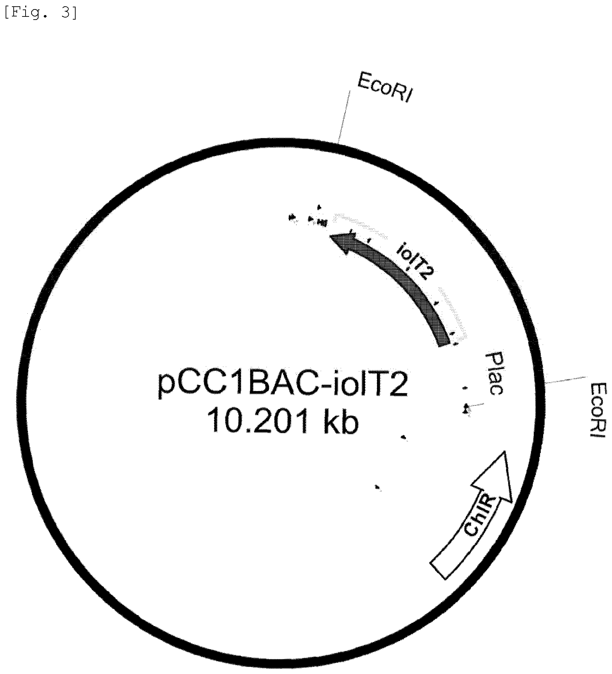

A recombinant vector comprising the *iolT2* gene of *Corynebacterium glutamicum* origin may be used. Preferably, the recombinant vector is pCC1BAC-*iolT2*. More preferably, the recombinant vector has a cleavage map shown in FIG. 3.

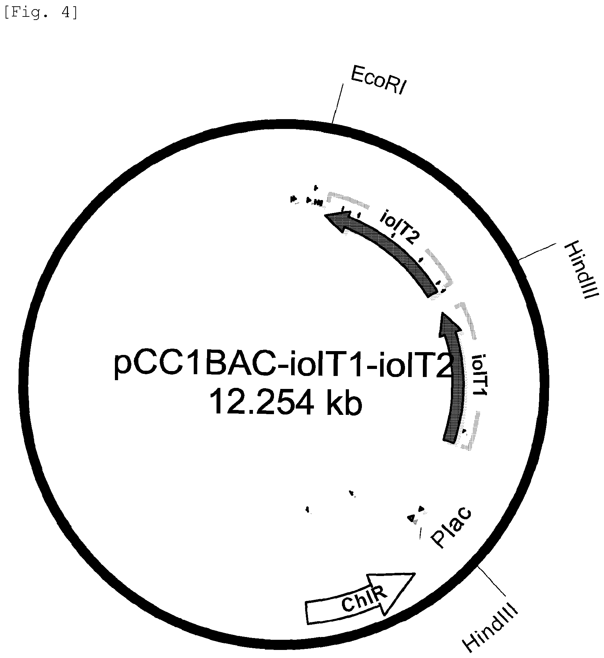

Preferably, the recombinant vector is for simultaneously expressing the *iolT1* and *iolT2* genes of *Corynebacterium glutamicum* origin. Preferably, the recombinant vector is pCC1BAC-*iolT1-iolT2.* More preferably, the recombinant vector has a cleavage map shown in FIG. 4.

As used herein, the term "transformation" means introducing a vector comprising a polynucleotide encoding a target protein into a host cell so as to be able to express a protein encoded by the polynucleotide in the host cell. The transformed polynucleotides include all the genes inserted in the chromosome of the host cell or located outside the chromosome, as long as they can be expressed in the host cell. In addition, the polynucleotides include DNA and RNA, which encode the target protein. As long as the polynucleotide can be introduced in the host cell and expressed therein, the gene may be introduced in any form. For example, the polynucleotide can be introduced into the host cell in the form of an expression cassette which is a polynucleotide construct including all elements for expressing the gene. The expression cassette includes a promoter which is operably linked to the gene, a transcription termination signal, a ribosome binding site, and a translation termination signal. The expression cassette may be in the form of an expression vector capable of self-replicating. The polynucleotide may also be introduced into the host cell by itself, and be operably linked to the sequence necessary for expression in the host cell.

In one embodiment, the present invention provides a microorganism of the genus *Escherichia* having enhanced rate of producing L-threonine, wherein the microorganism is obtained by transformation with a recombinant vector comprising the *iolT1* gene or *iolT2* gene of *Corynebacterium glutamicum* origin.

In an embodiment, the present invention provides a microorganism of the genus *Escherichia* having enhanced rate of producing L-threonine, wherein the microorganism is obtained by transformation with a recombinant vector comprising the *iolT1* gene and *iolT2* gene of *Corynebacterium glutamicum* origin.

Preferably, the transformed recombinant microorganism of the genus *Escherichia* may be *E. coli.* More preferably, the microorganism may be *E. coli* CA03-0230 (KCCM11370P), *E. coli* CA03-0260 (KCCM11369P) or *E. coli* CA03-0231 (KCCM11371P).

The recombinant, threonine-producing strains of the present invention, as described above, comprises the *iolT1* and/or *iolT2* gene of *Corynebacterium* origin introduced into *E*. *coli*, in which the introduced gene can increase the expression of permease in the *E. coli* strain to thereby greatly increase the sugar consumption rate and growth rate of the strain. Thus, the strains of the present invention can produce L-threonine at high concentration.

The present invention also provides a method for producing L-threonine, the method comprising the steps of: inoculating and culturing the transformed recombinant microorganism of the genus *Escherichia* of the invention; and separating L-threonine from the culture of the microorganism.

The recombinant microorganism of the genus *Escherichia* according to the present invention can be cultured by any conventional method. Specifically, the microorganism can be cultured by inoculating it into a medium that totally or partially contains sucrose or glucose as a carbon source. The culture process can be performed in suitable media and culture conditions known in the art. This culture process can be easily modified by any person skilled in the art depending on the type of strain selected. Examples of the culture process include, but are not limited to, batch culture, continuous culture, and fed-batch culture. The medium that is used in culture of the microorganism of the present invention should properly satisfy the requirements of the microorganism of the present invention.

Specifically, the medium that is used in the present invention contains sucrose or glucose as a main carbon source. Further, molasses containing a high concentration of sucrose may also be used as a carbon source. In addition, suitable amounts of various carbon sources may be used. Preferably, purified glucose is used. Examples of nitrogen sources that may be used in the present invention include organic nitrogen sources such as peptone, yeast extract, meat extract, malt extract, corn steep liquor, and soy meal, and inorganic nitrogen sources such as urea, ammonium sulfate, ammonium chloride, ammonium phosphate, ammonium carbonate, and ammonium nitrate. Preferably, peptone is used. These nitrogen sources may be used alone or in combination. The medium may contain potassium phosphate monobasic, potassium phosphate dibasic and corresponding sodium-containing salts, as phosphorus sources. Further, the medium may contain a metal salt such as magnesium sulfate or iron sulfate. In addition, the medium may contain amino acids, vitamins and suitable precursors. These media or precursors may be added to the medium in a batch or continuous manner.

The culture medium is typically maintained at a temperature ranging from 27°C to 37°C, and preferably from 30°C to 37°C. Culture of the microorganism can be continued until the desired level of the useful substance will be obtained. Preferably the culture period is from 10 to 100 hours.

Hereinafter, the present invention will be described in further detail with reference to examples. It is to be understood, however, that these examples are for illustrative purposes only and are not intended to limit the scope of the present invention.

### Examples

### Comparative Example: Construction of recombinant strains by transformation with recombinant vector comprising E. coli galP gene and comparison of L-threonine productivity

It was reported that galactose permease in *E. coli* functions as glucose permease, and when the expression of galactose permease is increased, for example, by increasing the copy number of galactose permease, the threonine productivity of the *E. coli* strain is increased (WO 2004/087937). To verify this report, a recombinant strain was constructed by increasing the copy number of the *galP* gene in the L-threonine-producing strain KCCM 10541, and the L-threonine productivity thereof was evaluated.

### (1) Construction of recombinant vector comprising E. coli galP gene

To obtain a 1.4-kb fragment comprising the open reading frame of the *E. coli galP* gene, the genomic DNA of the wild-type *E. coli* strain W3110 was extracted using a Genomic-tip system (Qiagen).

A polymerase chain reaction (hereinafter abbreviated as "PCR") was performed using the genomic DNA (gDNA) as a template. The PCR reaction was performed using primers of SEQ ID NOS: 9 and 10 under the following conditions: 30 cycles, each consisting of denaturation at 94°C for 30 sec, annealing at 56°C for 30 sec, and elongation at 72°C for 50 sec. The PCR product (hereinafter referred to as "galP fragment") was electrophoresed on 0.8% agarose gel, and then a band having a desired size was eluted.

The obtained *galP* fragment was treated with the restriction enzyme HindIII, and then ligated with a linear pCC1BAC vector (EPICENTRE, hereinafter the same) treated with the same restriction enzyme HindIII so that it would be in the same orientation with the lac promoter of the vector.

*E. coli* DH5a cells were transformed with the constructed vector, and then plated on a chloramphenicol-containing LB solid medium and cultured overnight at 37°C. One platinum loop of the cultured colony was inoculated into 3 mℓ of a chloramphenicol-containing LB liquid medium and cultured overnight, and then plasmid DNA was recovered using a plasmid miniprep kit (QIAGEN, hereinafter the same). The size of the recombinant vector was determined by treatment with the restriction enzyme HindIII (data not shown), and the clone was identified by performing PCR using primers of SEQ ID NOS: 11 and 12 under the following conditions: denaturation at 94°C for 30 sec, annealing at 56°C for 30 sec, and elongation at 72°C for 60 sec. The recombinant vector was named pCC1BAC-*galP* (data not shown).

In addition, the *galP* fragment was ligated with a linear pCL1920 vector treated with the same restriction enzyme HindIII so that it would be in the same orientation as the lac promoter of the vector. *E. coli* DH5a cells were transformed with the constructed vector, and then plated on a spectinomycin-containing LB medium and cultured overnight at 37°C. One platinum loop of the cultured colony was inoculated into 3 mℓ of a spectinomycin-containing LB liquid medium and cultured overnight, and then plasmid DNA was recovered using a plasmid miniprep kit. The size of the recombinant vector was determined by treatment with the restriction enzyme HindIII (data not shown), and the clone was identified by performing PCR using primers of SEQ ID NOS: 13 and 14 under the following conditions: denaturation at 94°C for 30 sec, annealing at 56°C for 30 sec, and elongation at 72°C for 60 sec. The recombinant vector was named pCL1920-*galP* (data not shown).

### (2) Construction of recombinant strain by transformation with recombinant vector

The recombinant vector pCC1BAC-*galP* was introduced into the L-threonine-producing strain *E. coli* KCCM10541 as a parent strain by electroporation, and the *E. coli* strain was plated on a solid medium containing 15 µg/mℓ of chloramphenicol to select a single colony. In the same manner as above, the recombinant vector pCL1920-galP was introduced into the L-threonine-producing strain *E. coli* KCCM10541 by electroporation, and the *E. coli* strain was plated on a solid medium containing 50 *µ*g/mℓ of spectinomycin to select a single colony.

The selected strains were named KCCM10541/pCC1BAC-*galP* and KCCM10541/pCL1920-*galP*, respectively.

### (3) Comparison of L-threonine productivity between recombinant strains

The recombinant strains constructed in the above section (2) were cultured using the threonine titer medium shown in Table 1 below in an Erlenmeyer flask according to the method described below, and the L-threonine productivities of the recombinant strains were examined.

**Table 1**

| Composition | Concentration (per liter) |
|---|---|
| Glucose | 70 g |
| KH₂PO₄ | 2 g |
| (NH₄)₂SO₄ | 27.5 g |
| MgSO₄·H₂O | 1 g |
| FeSO₄·H₂O | 5 mg |
| MnSO₄·H₂O | 5 mg |
| DL-methionine | 0.15 g |
| Yeast extract | 2 g |
| Calcium carbonate | 30 g |
| pH | 6.8 |

Titer evaluation was performed using the parent strain *E*. *coli* KCCM10541 and the KCCM10541/pCC1BAC-*galP* and KCCM10541/pCL1920-*galP* strains. When introduced into each of the strains, the recombinant vector pCC1BAC-*galP* is expressed as 1 copy, and the recombinant vector pCL1920-galP is expressed as 5 copies. The effect of the increase in the copy number was examined.

Each of the recombinant strains having different genetic characters was cultured overnight on LB solid medium in an incubator at 33°C, after which one platinum loop of each of the recombinant strains was inoculated into 25 mℓ of a glucose-containing titer medium having the composition shown in Table 1 above, and then was cultured in an incubator at 33°C and 200 rpm for 48 hours. The results of the culture are shown in Table 2 below.

**Table 2**

| Strain | L-threonine (g/L) | Sugar consumption rate (g/L/hr) |
|---|---|---|
| KCCM10541 | 32.0 | 0.753 |
| KCCM10541/pCC1BAC-*galP* | 31.1 | 0.793 |
| KCCM10541/pCL1920-*galP* | 30.8 | 0.816 |

As a result, as can be seen in Table 2 above, the parent strain *E. coli* KCCM10541 produced 32.0 g/L of L-threonine when cultured for 48 hours, but the recombinant strain *E. coli* KCCM10541/pCC1BAC-*galP* constructed in the Comparative Example produced 31.1 g/L of L-threonine, and the recombinant strain KCCM10541/pCL1920-*galP* produced 30.8 g/L of L-threonine. Thus, the L-threonine productivities of these recombinant strains were reduced by 0.9 g/L and 1.2 g/L, respectively, compared to that of the parent strain.

As can be seen in Table 2 above, the parent strain *E. coli* KCCM10541 showed a sugar consumption rate of 0.753 g/L/hr, but KCCM10541/pCC1BAC-*galP* showed a sugar consumption rate of 0.793 g/L/hr, and KCCM 10541/pCL1920-*galP* showed a sugar consumption rate of 0.816 g/L/hr. The sugar consumption rates of these recombinant strains increased by 5.3% and 8.4%, respectively, compared to that of the parent strain.

### Example 1: Construction of recombinant vector comprising iol T1 and/or iolT2 of Corynebacterium origin

### (1) Comparison of homology with E. coli glucose permease (galP)

It was reported that the *iolT1* and *iolT2* genes encoding inositol permease in *Corynebacterium glutamicum* has homology to the *galP* gene encoding *E. coli* glucose permease. Genes having homology to the *E. coli* galP gene were identified from the genome of wild-type *Corynebacterium glutamicum ATCC 13032* and compared, and the results of the comparison are shown in FIG. 1. The amino acid sequence encoded by *Corynebacterium glutamicum iolT1* showed a homology of 34% to the amino acid sequence encoded by E. coli *galP,* and the sequence encoded by *Corynebacterium glutamicum iolT2* showed a homology of 31% to the amino acid sequence encoded by E. coli *galP.*

### (2) Preparation of iolT1 gene fragment

To obtain a 1.5-kb fragment comprising the open reading frame of the *iolT1* gene of SEQ ID NO: 3, the genomic DNA of *Corynebacterium glutamicum ATCC 13032* was extracted using a Genomic-tip system (Qiagen).

A polymerase chain reaction was performed using the genomic DNA (gDNA) as a template. The PCR reaction was performed using primers of SEQ ID NOS: 5 and 6 under the following conditions: 30 cycles, each consisting of denaturation at 94°C for 30 sec, annealing at 56°C for 30 sec, and elongation at 72°C for 60 sec. The PCR product (hereinafter referred to as "*iolT1* fragment") was electrophoresed on 0.8% agarose gel, and then a band having a desired size was eluted.

### (3) Construction of recombinant vector pCC1BAC-iolT1

The *iolT1* fragment prepared in Example 1-(2) above was treated with the restriction enzyme HindIII, and then ligated with a linear pCC1BAC vector treated with the same restriction enzyme HindIII so that it would be in the same orientation with the lac promoter of the vector.

*E. coli* DH5a cells were transformed with the constructed vector, and then plated on a chloramphenicol-containing LB solid medium and cultured overnight at 37°C. One platinum loop of the cultured colony was inoculated into 3 mℓ of a chloramphenicol-containing LB liquid medium and cultured overnight, and then plasmid DNA was recovered using a plasmid miniprep kit. The size of the recombinant vector was determined by treatment with the restriction enzyme HindIII (data not shown), and the clone was identified by performing PCR using primers of SEQ ID NOS: 11 and 12 under the following conditions: denaturation at 94°C for 30 sec, annealing at 56°C for 30 sec, and elongation at 72°C for 90 sec. The recombinant vector was named pCC1BAC-*iolT1.*

### (4) Preparation of iolT2 gene fragment

To obtain a 1.6-kb fragment comprising the open reading frame of the *iolT2* gene of SEQ ID NO: 4, the genomic DNA of *Corynebacterium glutamicum ATCC 13032* was extracted using a Genomic-tip system (Qiagen).

A polymerase chain reaction was performed using the genomic DNA (gDNA) as a template. The PCR reaction was performed using primers of SEQ ID NOS: 7 and 8 under the following conditions: 30 cycles, each consisting of denaturation at 94°C for 30 sec, annealing at 56°C for 30 sec, and elongation at 72°C for 60 sec. The PCR product (hereinafter referred to as "*iolT2* fragment") was electrophoresed on 0.8% agarose gel, and then a band having a desired size was eluted.

### (5) Construction of recombinant vector pCC1BAC-iolT2

The *iolT2* fragment prepared in Example 1-(4) above was treated with the restriction enzyme EcoRI, and then ligated with a linear pCC1BAC vector treated with the same restriction enzyme EcoRI so that it would be in the same orientation with the lac promoter of the vector.

*E. coli* DH5a cells were transformed with the constructed vector, and then plated on a chloramphenicol-containing LB solid medium and cultured overnight at 37°C. One platinum loop of the cultured colony was inoculated into 3 mℓ of a chloramphenicol-containing LB liquid medium and cultured overnight, and then plasmid DNA was recovered using a plasmid miniprep kit. The size of the recombinant vector was determined by treatment with the restriction enzyme EcoRI (data not shown), and the clone was identified by performing PCR using primers of SEQ ID NOS: 11 and 12 under the following conditions: denaturation at 94°C for 30 sec, annealing at 56°C for 30 sec, and elongation at 72°C for 90 sec. The recombinant vector was named pCC1BAC-*iolT2*.

### (6) Construction of recombinant vector pCC1BAC-iolT1-iolT2

The pCC1BAC-*iolT2* vector constructed in Example 1-(5) above was treated with the restriction enzyme HindIII, and then ligated with the *iolT1* fragment prepared in Example 1-(2) above so that so that it would be in the same orientation with the lac promoter of the vector.

*E. coli* DH5a cells were transformed with the constructed vector, and then plated on a chloramphenicol-containing LB solid medium and cultured overnight at 37°C. One platinum loop of the cultured colony was inoculated into 3 mℓ of a chloramphenicol-containing LB liquid medium and cultured overnight, and then plasmid DNA was recovered using a plasmid miniprep kit. The size of the recombinant vector was determined by treatment with the restriction enzyme HindIII (data not shown), and the clone was identified by performing PCR using primers of SEQ ID NOS: 11 and 12 under the following conditions: denaturation at 94°C for 30 sec, annealing at 56°C for 30 sec, and elongation at 72°C for 120 sec. The recombinant vector was named *pCC1BAC-iolT1-iolT2.*

### Example 2: Construction of recombinant strains by transformation and comparison of L-threonine productivity

### (1) Construction of recombinant strains using wild-type E. coli

Each of the recombinant vectors (pCC1BAC-*iolT1*, pCC1BAC-*iolT2* and *pCC1BAC-iolT1-iolT2*) constructed in Example 1 above was introduced into wild-type *E. coli* MG1655 comprising the threonine operon-overexpressing vector pBRThrABCR3 (*Lee* KH et *al.*, Molecular Systems Biology (2007) 3:149) by electroporation, and then the *E. coli* cells were plated on solid media containing 100 µg/mℓ of ampicillin and 15 µg/mℓ of chloramphenicol to select single colonies.

The selected strains were named MG1655/pBRThrABCR3/pCC1BAC-*iolT1,* MG1655/pBRThrABCR3/pCC1BAC-*iolT2* and MG1655/pBRThrABCR3/pCC1BAC*iolT1-iolT2*, respectively.

The L-threonine productivities of these strains were analyzed in the same manner as described in Comparative Example 1-(3) above using the threonine titer medium having the composition shown in Table 1 above, and the results of the analysis are shown in Table 3 below.

**Table 3**

| Strain | L-threonine (g/L) | Sugar consumption rate (g/L/hr) |
|---|---|---|
| MG1655/pBRThrABCR3 | 3.86 | 0.877 |
| MG1655/pBRThrABCR3/pCC1BAC-iolT1 | 3.88 | 1.109 |
| MG1655/pBRThrABCR3/pCC1BAC-iolT2 | 3.92 | 1.035 |
| MG1655/pBRThrABCR3/pCC1BAC-iolT1-iolT2 | 3.85 | 1.123 |

As a result, as can be seen in Table 3 above, the parent strain *E. coli* MG1655 produced 3.86 g/L of L-threonine when cultured for 48 hours, and the recombinant strain MG1655/pCC1BAC-*iolT1* constructed in the Example of the present invention produced 3.88 g/L of L-threonine, and the recombinant strains MG1655/pCC1BAC-*iolT2* and MG1655/pCC1BAC-*iolT1-iolT2* produced 3.92 g/L and 3.85 g/L of L-threonine, respectively. In other words, the recombinant strains produced L-threonine at levels similar to that of the parent strain.

As can be seen in Table 3 above, the wild-type parent strain *E. coli* MG1655 showed a sugar consumption rate of 0.877 g/L/hr, but MG1655/pCC1BAC-*iolT2*, MG1655/pCC1BAC-*iolT1* and MG1655/pCC1BAC-*iolT1-iolT2* showed sugar consumption rates of 1.035 g/L/hr, 1.109 g/L/hr and 1.123 g/L/hr, which increased by 18.0%, 26.5% and 28.1%, respectively, compared to that of the parent strain.

### (2) Construction of recombinant strains using E. coli KCCM 10541

Each of the recombinant vectors (pCC1BAC-*iolT1*, pCC1BAC-*iolT2* and pCC1BAC-*iolT1-iolT2*) constructed in Example 1 above was introduced into the L-threonine-producing strain *E. coli* KCCM10541 as a parent strain by electroporation, and then the *E*. *coli* cells were plated on solid media containing 15 µg/mℓ of chloramphenicol to select single colonies.

The selected strains were named KCCM10541/pCC1BAC-*iolT1*, KCCM10541/pCC1BAC*-iolT2* and KCCM10541/pCC1BAC-*iolT1-iolT2,* respectively.

The L-threonine productivities of these strains together with the recombinant microorganisms obtained in Comparative Example 1-(2) above were analyzed in the same manner as described in Comparative Example 1-(3) using the threonine titer medium having the composition shown in Table 1 above, and the results of the analysis are shown in Table 4 below.

**Table 4**

| Strain | L-threonine (g/L) | Sugar consumption rate (g/L/hr) |
|---|---|---|
| KCCM 10541 | 30.3 | 0.823 |
| KCCM 10541/pCC1BAC-iolT1 | 29.5 | 1.213 |
| KCCM 10541/pCC1BAC-iolT2 | 32.5 | 1.093 |
| KCCM 10541/pCC1BAC-iolT1-iolT2 | 29.9 | 1.200 |
| KCCM 10541/pCC1BAC-galP | 29.8 | 0.862 |
| KCCM 10541/pCL1920-galP | 29.3 | 0.884 |

As a result, as can be seen in Table 4 above, the parent strain *E. coli* KCCM 10541 produced 30.3 g/L of L-threonine when cultured for 48 hours, and the recombinant strain KCCM 10541/pCC1BAC-*iolT2* constructed in Example 2-(2) above showed an increase in L-threonine production by 2.2 g/L compared to the parent strain, and KCCM10541/pCC1BAC-*iolT*1 and KCCM10541/pCC1BAC-*iolT1-iolT2* produced 29.5 g/L and 29.9 g/L of L-threonine, respectively. In other words, these recombinant strains produced L-threonine at levels similar to that of the parent strain.

The strains KCCM 10541/pCC1BAC-*galP* and KCCM 10541/pCL1920-*galP* showing an increased expression of the *E*. *coli galP* gene produced 29.8 g/L and 29.3 g/L of L-threonine, respectively.

As can be seen in Table 4 above, the parent strain *E. coli* KCCM 10541 showed a sugar consumption rate of 0.823 g/L/hr, but KCCM 10541/pCC1BAC-*iolT2*, KCCM 10541/pCC1BAC-*iolT1-iolT2* and KCCM 10541/pCC1BAC-*iolT1* showed sugar consumption rates of 1.093 g/L/hr, 1.200 g/L/hr and 1.213 g/L/hr, respectively, which increased by 32.8%, 45.8% and 47.4%, respectively, compared to that of the parent strain. In addition, because the sugar consumption rates of KCCM 10541/pCC1BAC-*galP* and KCCM 10541/pCL1920-galP increased by 4.7% and 7.4%, respectively, compared to that of the parent strain, it can be seen that the sugar consumption rates of the strains introduced with the *Corynebacterium iolT1* and/or *iolT2* gene significantly increased.

The transformed *E. coli* strains, KCCM 10541/pCC1BAC-*iolT1,* KCCM 10541/pCC1BAC*-iolT2* and KCCM 10541/pCC1BAC-*iolT1-iolT2,* were named CA03-0230, CA03-0260 and CA03-0231, respectively, and were deposited in the Korean Culture Center of Microorganisms; hereinafter abbreviated as 'KCCM') on February 5 , 2013 under accession numbers KCCM11370P, KCCM11369P and KCCM11371P, respectively.

The above-described results support that, when the expression of *Corynebacterium* permease in *E. coli* having L-threonine productivity is increased, that is, when one or more of the *iolT1* and *iolT2* genes are introduced into the *E. coli* strain, the sugar consumption rate increases, and the time required to produce the same concentration of L-threonine, that is, the threonine productivity increases, compared to when the expression of *E. coli* glucose permease in *E. coli* having L-threonine productivity is increased.

### Accession numbers

Depository authority: Korean Culture Center of Microorganisms;
Accession number: KCCM11370P;
Deposit date: February 5, 2013.
Depository authority: Korean Culture Center of Microorganisms;
Accession number: KCCM11369P;
Deposit date: February 5, 2013.
Depository authority: Korean Culture Center of Microorganisms;
Accession number: KCCM11371P;
Deposit date: February 5, 2013.

## Claims

1. A recombinant microorganism of the genus *Escherichia* having enhanced rate of producing L-threonine, wherein the recombinant microorganism is obtained by transformation so as to contain a permease of *Corynebacterium* origin represented by SEQ ID NO: 1 or SEQ ID NO: 2, or a permease having at least 80% identity to SEQ ID NO: 1 or SEQ ID NO: 2.

2. The recombinant microorganism of the genus *Escherichia* having enhanced rate of producing L-threonine, according to claim 1, wherein the recombinant microorganism is *Escherichia coli.*

3. The recombinant microorganism of the genus *Escherichia* according to claim 1, wherein the recombinant microorganism is obtained by transformation so as to contain both the *Corynebacterium* permeases of SEQ ID NO: 1 and SEQ ID NO: 2, or permeases having at least 80% identity to SEQ ID NO: 1 and SEQ ID NO: 2.

4. A method for producing L-threonine, comprising the steps of:
inoculating and culturing the microorganism of any one of claims 1 to 3; and
separating L-amino acid from the culture.

## Patentansprüche

1. Rekombinanter Mikroorganismus der Gattung Escherichia, der eine erhöhte L-Threonin-Produktionsrate aufweist, wobei der rekombinante Mikroorganismus durch Transformation erhalten wird, sodass er eine Permease von Corynebacterium, dargestellt durch SEQ ID NO: 1 oder SEQ ID NO: 2, oder eine Permease, die mindestens 80 % Identität zu SEQ ID NO: 1 oder SEQ ID NO: 2 aufweist, enthält.

2. Rekombinanter Mikroorganismus der Gattung Escherichia, der eine erhöhte L-Threonin-Produktionsrate aufweist, nach Anspruch 1, wobei der rekombinante Mikroorganismus Escherichia coli ist.

3. Rekombinanter Mikroorganismus der Gattung Escherichia nach Anspruch 1, wobei der rekombinante Mikroorganismus durch Transformation erhalten wird, sodass er sowohl die Corynebacterium-Permeasen von SEQ ID NO: 1 als auch SEQ ID NO: 2 oder Permeasen, die mindestens 80 % Identität zu SEQ ID NO: 1 und SEQ ID NO: 2 aufweisen, enthält.

4. Verfahren zum Herstellen von L-Threonin, umfassend die Schritte von:
Animpfen und Kultivieren des Mikroorganismus nach einem der Ansprüche 1 bis 3; und
Trennen von L-Aminosäure aus der Kultur.

## Revendications

1. Micro-organisme recombinant du genre Escherichia présentant un taux accru de production de L-thréonine, dans lequel le micro-organisme recombinant est obtenu par transformation de manière à contenir une perméase d'origine Corynebacterium représentée par SEQ ID NO : 1 ou SEQ ID NO : 2, ou une perméase présentant au moins 80 % d'identité avec SEQ ID NO : 1 ou SEQ ID NO : 2.

2. Micro-organisme recombinant du genre Escherichia présentant un taux accru de production de L-thréonine, selon la revendication 1, dans lequel le micro-organisme recombinant est Escherichia coli.

3. Micro-organisme recombinant du genre Escherichia selon la revendication 1, dans lequel le micro-organisme recombinant est obtenu par transformation de manière à contenir à la fois les perméases Corynebacterium de SEQ ID NO : 1 et SEQ ID NO : 2, ou des perméases présentant au moins 80 % d'identité avec SEQ ID NO : 1 et SEQ ID NO : 2.

4. Procédé de production de L-thréonine, comprenant les étapes de :
inoculation et culture du micro-organisme selon l'une quelconque des revendications 1 à 3 ; et
séparation de l'acide L-aminé de la culture.
